# FASCICULE DE BREVET EUROPEEN

(11) **EP 1 960 545 B1**
(45) Date de publication et mention de la délivrance du brevet: **16.04.2014**
(21) Numéro de dépôt: 06830607.5
(22) Date de dépôt: 13.12.2006
(51) Int. Cl.: C12Q 1/68

(54) **PROCEDE D'AUTOCALIBRATION DE BIOPUCES**
BIOCHIP-SELBSTKALIBRIERUNGSPROZESS
BIOCHIP SELF-CALIBRATION PROCESS

(30) Priorité: 13.12.2005 FR 0512594
(43) Date de publication de la demande: 27.08.2008
(73) Titulaire: CENTRE NATIONAL DE LA RECHERCHE SCIENTIFIQUE (CNRS), 75016 Paris (FR); Université Paris Sud, 91400 Orsay (FR)
(72) Inventeur: CANVA, Michael, Thomas, Georges, F-91190 Gif S/Yvette (FR)
(74) Mandataire: Regimbeau
(86) Numéro de dépôt international: PCT/EP2006/069683
(87) Numéro de publication internationale: WO 2007/068725

(56) Documents cités:
- EP-A- 1 065 280
- WO-A-02/18655
- WO-A-03/100077
- US-B1- 6 423 535
- DATABASE WPI Week 200405 Derwent Publications Ltd., London, GB; AN 2004-053308 XP002395084 & WO 03/100422 A1 (JAPAN GENOME SOLUTIONS INC) 4 décembre 2003 (2003-12-04)
- DATABASE WPI Week 200473 Derwent Publications Ltd., London, GB; AN 2004-748765 XP002395085 & WO 2004/090129 A1 (HITACHI HIGH TECHNOLOGIES CORP) 21 octobre 2004 (2004-10-21)

## Description

La présente invention a pour objet un procédé de calibration interne pour la détermination de la présence et/ou de la quantité d'un composé cible dans un échantillon à tester, ledit procédé mettant en oeuvre un support solide sur lequel est fixé à sa surface supérieure un composé sonde de calibration, notamment par imagerie SPR ou par fluorescence. La présente invention comprend également un kit et un dispositif pour la mise en oeuvre d'un tel procédé.

De nombreuses techniques ou dispositifs d'analyse d'échantillons biologiques ont été développés ces dernières années, en particulier pour l'analyse en parallèle de grandes quantités d'acides nucléiques ou de protéines, notamment suite à l'essor de la génomique ou de la protéomique. Ainsi, la technologie des puces à ADN ou à protéines ou biopuces, connaît à l'heure actuelle un essor exceptionnel et suscite un formidable intérêt dans la communauté scientifique. La connaissance du niveau d'expression d'un gène dans ces différentes situations constitue une avancée vers sa fonction, mais également vers le criblage de nouvelles molécules et l'identification de nouveaux médicaments et de nouveaux outils de diagnostic.

Parmi ces techniques ou dispositifs, on peut citer les supports permettant de réaliser l'analyse à haut débit d'acides nucléiques ou de peptides, tels que les biopuces, ou puces à ADN ou à protéines (dénommés aussi « micro- ou macroarrays », ou encore « DNA chip ») ont fait l'objet de nombreuses études.

Ces biopuces peuvent être en particulier réalisées à partir d'un support solide, fonctionnalisé sur lequel ont été fixés et localisés des acides nucléiques ou peptides donnés (sondes nucléiques ou peptidiqués) et sur lesquelles sondes nucléiques ou peptidiques vont se fixer spécifiquement respectivement par appariement (ou hybridation spécifique) ou par reconnaissance d'un site d'affinité les acides nucléiques ou protéines cibles que l'on cherche à détecter, identifier et/ou quantifier dans l'échantillon biologique.

Parmi les documents décrivant les techniques relatives aux biopuces à ADN, on peut citer en particulier :
- l'article de revue de Wang J. (Nucleic Acids Research, 28, 16, 3011-3016, 2000), qui présente un résumé faisant le point sur les principales techniques connues relatives aux puces à ADN, et le document Schubhart et al. (Nucleic Acids Research, 28, 10, e47, 2000) qui dresse une liste des problèmes auxquels sont confrontés les concepteurs de ces puces ;
- le document brevet délivré sous le N° US 6,030,782, qui décrit un greffage avec une surface mercaptosilanisée, d'acides nucléiques modifiés par un groupe sulhydryle ou disulfure, et l'article de Bamdad (Biophysical Journal, 75, 1997-2003, 1998), qui décrit l'obtention de surfaces présentant des ADN par incorporation de molécules composites, les ADN-thiols, dans des monocouches auto-assemblées (« self-assembled monolayers ou SAMs ») ;
- la demande internationale de brevet publiée sous le N° WO 00/43539 qui propose d'immobiliser des molécules, telles que des oligonucléotides, par le biais de polymères polyfonctionnels ce qui permet d'augmenter la densité de greffage. Ces polymères peuvent être obtenus à partir de monomères tels que le méthacrylate d'hydroxyéthyle, d'acrylamide, de vinyl pyrrolidone ;
- la demande internationale de brevet publiée sous le N° WO 00/36145 décrit de son côté une méthode de fabrication de puces à ADN, comprenant la polymérisation sur un substrat de type couche métallique, d'un copolymère de pyrrole et de pyrrole fonctionnalisé, la fixation d'un agent de réticulation sur le pyrrole fonctionnalisé, puis la fixation d'une sonde biologique (telle qu'un oligonucléotide). L'agent de réticulation peut être bifonctionnel, et par exemple présenter une fonction ester de la N-hydroxysuccinimide et une fonction maléimide ;
- la demande internationale de brevet publiée sous le N° WO 98/20020 qui décrit également l'immobilisation à haute densité d'acides nucléiques sur des supports solides, cette fois-ci par mise en contact d'un acide nucléique contenant un groupement thiol avec un support présentant un groupe réagissant avec ce thiol, éventuellement par l'intermédiaire d'un agent de réticulation.

Parmi les techniques ou dispositifs d'analyse d'échantillons biologiques qui ont été également développés ces dernières années pour l'analyse en parallèle d'acides nucléiques ou de protéines, on peut également citer l'imagerie par résonance plasmonique de surface, dénommée SPR pour « Surface Plasmon Resonance », technique qui au cours des dernières années est devenue un outil analytique bien accepté pour le suivi des processus interfaciaux tout comme la caractérisation de films minces. On peut noter qu'une telle technique a pour avantage de ne pas nécessiter de marquage du complexe spécifique formé entre la sonde fixée sur le support et le composé cible que l'on cherche à détecter, identifier et/ou quantifier. Dans cette technique de SPR, la sensibilité de la méthode provient de la stimulation des champs électromagnétiques, ondes plasmons de surface, créés sur une interface métal-diélectrique. Cette localisation du champ lumineux au voisinage immédiat de l'interface limite les interactions lumière:matière à un faible volume dont les moindres modifications auront des conséquences importantes sur les propriétés des plasmons de surface. De tels plasmons surfaciques sont excités notamment sur des surfaces d'or quand une lumière polarisée TM illumine l'interface or/diélectrique par un prisme sous réflexion totale, couplant sous un certain angle la lumière incidente dans les modes de plasmons de surface, ou via un réseau de diffraction. La formation du plasmon est corrélée à une diminution marquée de la lumière réfléchie telle que peut être mesurée par une photodiode. Les modifications de l'état de surface, notamment celles liées aux fonctionnement normal d'une biopuce entraîneront des modifications des conditions d'existence des ondes plasmons de surface et donc de leur efficacité de couplage dans une configuration optique donnée. On peut ainsi quantifier les cibles s'hybridant notamment en mesurant la modification d'angle ou de longueur d'onde de résonance, voire même simplement la variation de réflectivité. Ces changements seront extrêmement sensibles à tout changement dans l'indice de réfraction (n) du milieu adjacent et tout changement de l'épaisseur optique. L'or et l'argent sont par exemple les candidats idéaux en tant que films métalliques pour une puce de SPR dans la région de la lumière visible. La technique de SPR a été largement utilisée pour la détection sans marquage, l'étude de la réaction d'hybridation de l'ADN et la détection des événements moléculaires et biomoléculaires en temps réel. Ceci est possible du fait que le principe de détection est basé sur le changement de contraste optique induit par une molécule liée à l'interface en comparaison au milieu environnant. La chimie utilisée pour l'immobilisation des composants biologiques à la, surface d'or de la puce de SPR est principalement basée sur l'utilisation de composés thiolés (voir par exemple Peterlinz K. A. et al., Am. Chem. Soc., 1997, 119, 3401-3402 ; Smith E. A. et al., Langmuir, 2001, 17, 2502-2507 ; Smith E. A. et al., Am. Chem. Soc., 2003, 125, 6140-6148 ; Damos F. S. et al., Langmuir, 2005, 21, 602-609) ou des polymères conducteurs (voir par exemple Guedon P. et al., Anal. Chem., 2000, 72, 6003-6009 ; Jung L. S. et al., J. Phys. Chem. B., 2000, 104, 11168-11178 ; Szunerits S. et al., Langmuir, 2004, 20, 9236-9241). L'entreprise Biacore fabrique des systèmes bioanalytiques basés sur le phénomène de SPR (Voir : http//www.biacore.com.). Dans ce système, on a utilisé une couche de dextrane fonctionnalisée couplée à la surface d'or pour lier différentes espèces chimiques et biologiques sur la surface.

Dans le contexte des technologies post-génomiques, il est urgent de disposer de systèmes de biopuces de routine à l'extérieur des laboratoires de recherche. Avant d'y parvenir, il est nécessaire de mieux caractériser et comprendre les matériaux et composants actuellement mis en oeuvre afin d'améliorér les qualités et les performances de systèmes de biopuces.

Actuellement, les plateformes techniques utilisant ces biopuces sont pour la plupart installées dans les laboratoires de recherche. En effet, l'interprétation des mesures obtenues nécessite encore l'intervention de spécialistes, ceci dû au grand niveau de « bruit » et/ou de « fluctuation » associé à ces mesures ce qui rend leur interprétation quantitative difficile. Dans le cadre particulier du diagnostic génétique, il s'ensuit un manque de fiabilité dans le génotypage effectué à grande échelle, sous forme automatisée, prohibant jusqu'à présent leur utilisation de routine à bas coût. Actuellement, le besoin n'est donc pas comblé.

La précision des mesures est actuellement limitée par la maîtrise relative de la fonctionnalité des surfaces mises en jeu. Dans le cas d'utilisation de plots de ces biopuces (un plot, ou spot, correspondant à une surface distincte de la puce où sont déposées les mêmes entités), ceci se traduit spatialement par une absence d'homogénéité de chacun d'entre eux et par une grande hétérogénéité entre eux. Il arrive même que les différences que l'on cherche à distinguer soient plus petites que ces dispersions intrinsèques aux surfaces des biopuces.

Ainsi, il reste à pouvoir disposer d'une méthode permettant d'autocalibrer chacun des pixels des images obtenues afin d'améliorer significativement la mesure de la réactivité des interactions biochimiques dont ils sont les lieux.

Une telle méthode devrait impliquer un très faible surcoût dans la préparation des biopuces et dans leur protocole d'utilisation tout en permettant un gain significatif de leur précision, et par la même à terme de leur densité d'information à performances égales.

Ceci est justement l'objet de la présente invention.

Les inventeurs ont mis en évidence qu'il était possible, notamment dans le cas des biopuces à ADN, d'introduire dans un même plot, et le cas échéant dans plusieurs plots, en plus du composé sonde (Cs) spécifique du composé cible (Cc) à analyser un composé sonde de calibration (Csc) commun spécifique d'un composé cible de calibration (Ccc). Un seul passage de solution contenant ce composé Ccc permet de quantifier la densité effective des sondes Cs accessibles sur chacun des pixels, valeur qui sera utilisée comme paramètre individualisé de formalisation. Avec un tel procédé, les inventeurs ont mis en évidence qu'il était possible de diminuer significativement la dispersion des mesures effectuées sur chacun des pixels d'un même plot, ou entre plusieurs plots complémentaires, aboutissant ainsi à l'amélioration et à une plus grande précision des mesures associées à ces biopuces.

Avec un tel procédé, les inventeurs ont mis en évidence qu'il était possible de réaliser l'autocalibration de la mesure obtenue pour chacun des pixels de la biopuce permettant ainsi de s'affranchir de la plupart des fluctuations induites sur les mesures brutes par lès inhomogénéités des matériaux et composants mis en jeu.

Le procédé d'auto-calibration de biopuces selon l'invention repose sur:
- la lecture séquentielle par une méthode dynamique de lecture des réactions de formation des complexes sonde:cible sur une surface préalablement fonctionnalisée et structurée ; et
- la fonctionnalisation adéquate de cette surface par des sondes multi-fonctionnalisées comprenant au moins une sonde de calibration et une sonde spécifique.

Ainsi, la présente invention a pour objet un procédé d'autocalibration d'une mesure effectuée sur un support solide (biopuce) destinée à déterminer la présence et/ou la quantité d'un composé cible Cc dans un échantillon à tester, ledit procédé mettant en oeuvre un support solide sur lequel est fixé à sa surface supérieure un composé sonde Cs capable de se lier spécifiquement avec ledit composé Cc susceptible d'être contenu dans l'échantillon à tester, ledit procédé comprenant :
- une étape a) de mise en contact de l'échantillon à tester susceptible de contenir ledit composé Cc avec ledit support dans des conditions permettant la formation spécifique du complexe Cc/Cs ; et
- une étape b) de mesure de la formation du complexe spécifique Cc/Cs éventuellement formé à l'étape a), de préférence par une méthode de mesure dynamique sans marqueur, caractérisé en ce que :

- ledit support solide comprend en outre fixé à cette surface supérieure un composé sonde de calibration Csc capable de se lier spécifiquement avec un composé cible de calibration Ccc, le rapport molaire entre ledit composé Csc et ledit composé Cs étant connu, et en ce que le procédé comprend préalablement ou postérieurement aux étapes a) et b) les étapes suivantes :
   c) la mise en contact d'un échantillon dudit composé Ccc avec ledit support dans des conditions permettant la formation spécifique du complexe Csc/Ccc ; et
   d) la mesure de la formation du complexe Csc/Ccc à l'étape c) par la méthode de mesure utilisée à l'étape b),
la détermination de la présence et/ou de la quantité dudit composé cible Cc dans l'échantillon à tester étant basée sur les mesures obtenues à l'étape b) et à l'étape d).

Les conditions permettant la formation spécifique de complexe Cc/Cs ou Ccc/Csc, notamment de complexe spécifique acide nucléique/acide nucléique ou polypeptide/polypeptide sont bien connues de l'art et ne seront pas développées ici. Par exemple, lorsqu'il s'agit de complexes d'acide nucléique, on entend désigner ici les conditions permettant l'hybridation spécifique desdits acides nucléiques cibles avec lesdits acides nucléiques sondes. Il s'agit de préférence de conditions de forte stringence notamment telles que définies ci-après.

Une hybridation dans des conditions de forte stringence signifie que les conditions de température et de force ionique sont choisies de telle manière qu'elles permettent le maintien de l'hybridation entre deux fragments d'ADN ou d'ARN/ADN complémentaires. A titre illustratif, des conditions de forte stringence de l'étape d'hybridation aux fins de définir les conditions d'hybridation décrites ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook et al. (1989, Molecular cloning: a laboratory manual. 2nd Ed. Cold Spring Harbor).

Pour les complexes Cc/Cs ou Ccc/Csc de type polypeptide/polypeptide, il s'agit le plus souvent de complexe spécifique obtenu par affinité entre les deux polypeptides (type ligand/récepteur, antigène/anticorps, etc.). Les conditions permettant d'obtenir la formation de tels complexes d'affinité sont également bien connues, notamment en terme de température d'incubation (entre 20 et 37°C), de la molarité, pH et/ou salinité des tampons utilisés (type tampon PBS à pH 7, 4).

Selon un mode de réalisation préféré, le procédé selon l'invention est caractérisé en ce que :
- lorsque les étapes c) et d) sont réalisées préalablement aux étapes a) et b), le support solide est lavé dans des conditions appropriées après l'étape d) afin d'éliminer du support solide le composé Ccc du complexe Csc/Ccc ; ou
- lorsque les étapes c) et d) sont réalisées postérieurement aux étapes a) et b), le support solide est lavé dans des conditions appropriées après l'étape b) afin d'éliminer du support solide le composé Cc du complexe Cs/Cc.

Selon la nature du complexe obtenu (acide nucléique/acide nucléique, polypeptide/polypeptide ou encore acide nucléique/polypeptide), l'homme de l'art connaît les conditions et solutions à appliquer permettant de dénaturer un tel complexe.

Par exemple et sans s'y limiter on pourra utiliser une solution de rinçage à environ 80°C pour dénaturer un duplex d'acide nucléique formé pour le complexe Csc/Ccc ou Cs/Cc. On pourra également utiliser les conditions de dénaturation utilisées habituellement pour obtenir un composé sonde d'acide nucléique Cs de type simple brin à partir de composé sonde de type double brin fixé au support (voir par exemple Peterson et al., Nucleic Acids Research, 29 (24), 5163-5168, 2001, Materials and Method).

Pour les complexes de type polypeptide/polypeptide, l'élimination du composé Cc ou Ccc du complexe formé pourra être réalisée avec des solutions de rinçage dont le pH et/ou la force ionique sera convenablement ajusté pour obtenir la dénaturation du complexe. De telles méthodes sont couramment utilisées en méthode de chromatographie d'affinité pour éluer les polypeptides d'intérêt complexés par affinité à un autre polypeptide fixé au support de chromatographie.

Si le lavage du support solide après l'étape d) ou b) afin d'éliminer du support solide le composé Ccc ou Cc respectivement du complexe Csc/Ccc ou Cs/Cc peut être avantageusement utilisé pour tout type de système de mesure, il est particulièrement avantageux d'effectuer un tel lavage lorsque l'on souhaite utiliser un système de mesure nécessitant un marquage des composés cibles Cc ou Ccc en utilisant un même marqueur.

Selon un mode de réalisation préféré, le procédé selon l'invention est caractérisé en ce que les étapes c) et d) sont réalisées préalablement à l'étape a).

Il peut être avantageux, en particulier lorsque les composés sondes Cs et Csc sont des acides nucléiques, de fixer entre le support et les composés sondes des bras espaceurs sur lesquels seront fixés les composés sondes, pour donner de la mobilité à la sonde greffée. En effet, pour en particulier les oligonucléotides de courte séquence, les acides nucléiques greffés au support (par adsorption ou par couplage covalent) ne sont pas toujours entièrement accessibles à la séquence cible lors de l'hybridation. Le composé espaceur est généralement fixé à l'extrémité du composé sonde initialement prévu pour être fixé au support. La nature de ce composé peut être variable telle qu'une séquence d'acide nucléique n'ayant aucune homologie avec les séquences cibles Cc ou Ccc ou leurs séquences complémentaires ou encore des composés de type poly(éthylène glycol) (voir document brevet WO 03/068712).

Ainsi, selon un mode de réalisation préféré le procédé selon l'invention est caractérisé en ce qu'un composé espaceur est fixé sur ledit support, et en ce que le composé Cs et le composé Cc sont fixés au support, indépendamment l'un par rapport à l'autre par l'intermédiaire dudit composé espaceur.

Selon un mode de réalisation préféré, le procédé selon l'invention est caractérisé en ce que le composé Csc est fixé au composé Cs par liaison covalente.

Selon un mode de réalisation préféré, le procédé selon l'invention est caractérisé en ce que le composé Csc est fixé à l'extrémité libre non fixée au support du composé Csc.

Selon un mode de réalisation préféré, le procédé selon l'invention est caractérisé en ce que le composé Csc est fixé à la surface du support solide indépendamment du composé Cs, le rapport molaire entre ledit composé Csc fixé et ledit composé Cs fixé étant connu.

Par indépendamment, on entendra désigner ici que le composé Csc n'est pas fixé directement sur le composé Cs.

Selon un mode de réalisation préféré, le procédé selon l'invention est caractérisé en ce que le composé Csc et le composé Cs sont fixés à la surface du support solide par fixation à une même molécule, cette dernière étant fixée au support.

Selon un mode de réalisation préféré, le procédé selon l'invention est caractérisé en ce que la même molécule sur laquelle sont fixés le composé Csc et le composé Cs est un polymère tel que par exemple mais sans s'y limiter un polymère de pyrrole ou un poly(éthylène-imine) couplé à plusieurs avidines.

On pourra par exemple pour les systèmes utilisant une mesure par imagerie SPR se référer à la méthode de fixation par électropolymérisation de résidus pyrrole portant un composé sonde (Cs ou Csc) sur une lame de verre revêtue d'une surface métallique (Maillart et al., Oncogene, p. 1-8, 2004, et Guedon et al., 2000) ou la méthode mettant en oeuvre du poly(éthylène-imine) couplé avec de l'avidine avec des composés sondes biotinylés (Bassil et al., Sensors and Actuators, B 94, 313-323, 2003).

Selon un mode de réalisation préféré, le procédé selon l'invention est caractérisé en ce que :
- les mêmes composés Cs et Csc sont fixés sur une même surface délimitée (dénommée « plot » ou « spot ») du support ;
- en ce que l'échantillon du composé Ccc et l'échantillon à tester susceptible de contenir le composé Cc à l'étape c) sont mis en contact avec tout ledit plot du support
- on effectue les mesures de l'étape b) et de l'étape d) pour un ensemble de points ou pixels dudit plot ; et
- la détermination de la présence et/ou de la quantité dudit composé cible Cc dans l'échantillon à tester est réalisée en tenant compte de l'ensemble des mesures obtenues à l'étape b) et à l'étape d) pour chacun des points ou pixels mesurés dudit plot.

Selon un mode de réalisation préféré, le procédé selon l'invention est caractérisé en ce que pour un même plot, les conditions permettant la formation spécifique du complexe Cc/Cs et Ccc/Csc sont identiques.

Selon un mode de réalisation préféré, le procédé selon l'invention est caractérisé en ce que ledit support comprend n plots, n étant compris entre 2 et 10⁸, de préférence entre 2 et 10⁷, entre 2 et 10⁶, entre 2 et 10⁵, entre 2 et 10⁴, entre 2 et 10³ ou entre 2 et 10³ plots.

Selon un mode de réalisation préféré, le procédé selon l'invention est caractérisé en ce que les composés Csc et Ccc utilisés sont identiques pour les n plots.

Selon un mode de réalisation préféré, le procédé selon l'invention est caractérisé en ce que les composés Csc et Ccc utilisés sont différents pour au moins 2 plots.

Selon un mode de réalisation préféré, le procédé selon l'invention est caractérisé en ce que les composés Cs utilisés et les composés Cc recherchés sont différents entre au moins 2 plots.

Selon un mode de réalisation préféré, le procédé selon l'invention est caractérisé en ce que les conditions permettant la formation spécifique du complexe Cc/Cs ou Ccc/Csc sont identiques pour les 2 plots entre lesquels le composé Cs utilisé et le composé Cc recherché sont différents, de préférence entre tous les plots du support solide.

Selon un mode de réalisation préféré, le procédé selon l'invention est caractérisé en ce que ledit composé Cs est choisi parmi le groupe de composés constitué par les acides nucléiques, les peptides-nucléiques acides (PNA), les polypeptides, les oligosaccharides, les lipides, de préférence les acides nucléiques, les PNA et les polypeptides.

Selon un mode de réalisation préféré, le procédé selon l'invention est caractérisé en ce que ledit composé Cs et ledit composé Cc sont choisis par les couples (Cs, Cc) suivants :
- (acide nucléique, acide nucléique) ;
- (acide nucléique, polypeptide) ;
- (polypeptide, acide nucléique) ; et
- (polypeptide, polypeptide),
le couple (Cs, Cc) (acide nucléique, acide nucléique) étant le couple préféré.

Selon un mode de réalisation préféré, le procédé selon l'invention est caractérisé en ce que :
- lorsque ledit composé Cs et ledit composé Cc sont des acides nucléiques, les composés Csc et Ccc sont des acides nucléiques ;
- lorsque ledit composé Cs et ledit composé Cc sont des polypeptides, les composés Csc et Ccc sont des polypeptides ; et
- lorsque ledit composé Cs est un acide nucléique et ledit composé Cc est un polypeptide, le composé Csc est un acide nucléique.

Selon un mode de réalisation préféré, le procédé selon l'invention est caractérisé en ce que ledit composé Cs et ledit composé Cc sont des acides nucléiques, notamment choisis parmi le groupe d'acides nucléiques comprenant les acides nucléiques de type ADN double brin, ADN simple brin ou encore de type mixte simple et double brin, les produits de transcription desdits ADNs, tels que les ARNs, ainsi que tout acide nucléique comportant ou non des nucléotides non naturels.

Selon un mode de réalisation préféré, le procédé selon l'invention est caractérisé en ce que le composé Csc fixé à un plot du support solide un acide nucléique ne présentant aucune homologie significative avec le composé Cs fixé et le composé Cc recherché, ou leur séquence complémentaire, sur ce plot.

Par aucune homologie significative, on entendra désigner ici un pourcentage d'identité entre les séquences tel qu'il ne permet pas l'hybridation entre ces séquences dans les conditions d'hybridation spécifique utilisées lors des étapes a) de mise en contact de l'échantillon à tester susceptible de contenir ledit composé Cc avec ledit support dans des conditions permettant la formation spécifique du complexe Cc/Cs, et de l'étape c) de mise en contact d'un échantillon dudit composé Ccc avec ledit support dans des conditions permettant la formation spécifique du complexe Csc/Ccc.

Selon un mode de réalisation préféré, le procédé selon l'invention est caractérisé en ce que le composé Csc et, le cas échéant, le composé Ccc, est un acide nucléique de longueur comprise entre 6 et 30 nucléotides, de préférence entre 6 et 24 nucléotides, entre 6 et 20 nucléotides, entre 6 et 15 nucléotides ou entre 6 et 12 nucléotides.

Selon un mode de réalisation préféré, le procédé selon l'invention est caractérisé en ce que ledit support est un support solide de préférence choisi parmi les supports en verre, en silicium, en silicone, Kevlar™, en polymère (tel que le plastique, le polyacrylamide, le polypyrrole, des polyoses), les métaux (or, platine). Lorsque le système de mesure utilisé est l'imagerie par SPR, ces supports, notamment en verre, seront revêtus à l'une de leurs surfaces d'une fine couche métallique (par exemple en or ou argent).

Ces supports ainsi que les méthodes de fixation des composés sondes Cs et Csc sont bien connus de l'homme de l'art.

Parmi ces supports on peut citer notamment ceux nécessitant un marquage des cibles pour leur détection comme par exemple :
- Les supports de type « Macroarray »
   Support : membrane de nylon ; taille des spots : 0,5-1 mm ; densité : quelques centaines de spots/cm² ; sondes : par exemple produits de PCR ou de synthèse ; cibles : par exemple ADNc avec marquage radioactif au ³²P ;
- Les supports de type « Microarray spottée »
   Support : lame de verre à revêtement chimique ; taille des spots : ∼100 µm ; densité : 1000-10000 spots/cm² ; sondes : par exemple produits de PCR ou oligonucléotides de synthèse (20-70 mers) ; cibles : par exemple ADNc ou produits de PCR avec marquage fluorescent (au Cy3 et Cy5) ;
- Les supports de type « GeneChips » de Affymetrix
   Support : lame de verre à revêtement chimique ; taille des spots : ∼20 µm ; densité : jusque 250000 spots/cm²; sondes : par exemple des oligonucléotides de 20-25 mers synthétisés *in situ ;* cibles : ARNc ou produits de PCR avec marquage fluorescent à la biotine-streptavidine.

Les méthodes de fabrication des puces « spottées » sont aujourd'hui bien établies (DeRisi et al., Science, 278(5338):p. 680-686, 1997). Lorsque les composés cibles sont des acides nucléiques de type ADN, les solutions d'ADN sont préparées soit par amplification PCR, soit par synthèse d'oligonucléotides, des micro-gouttelettes de ces solutions sont ensuite déposées par un robot, selon une matrice d'emplacements définis, sur une lame de verre traitée par un revêtement chimique qui permet de fixer l'ADN sonde sur chaque spot (ou plot) de la matrice.

Les méthodes de fabrication des puces à oligonucléotides synthétisés *in situ* par photolithographie sont également bien établies ; on pourra se référer à la technologie « GeneChips™ » de la société Affymetrix (Lipshutz R.J. et al., Nat. Genet., 1999, 21(1 Suppl):p.20-24) ou celle par impression jet d'encre d'Agilent Technologies/Rosetta Inpharmaceutics (Hughes T.R. et al., Nat. Biotechnol, 2001, 19(4):p. 342-347).

Dans le procédé d'autocalibration de biopuces selon la présente invention, la lecture à l'étape b) et d) peut être réalisée par toute méthode de lecture dynamique, de préférence par toute méthode de lecture dynamique sans marqueur.

Parmi ces méthodes de lecture dynamiques sans marqueur qui peuvent être utilisées dans le procédé d'autocalibration de biopuces selon la présente invention, on peut citer, sans toutefois s'y restreindre :
- l'imagerie par résonance de plasmons de surface (Surface Plasmon Résonance (« SPR »)) ;
- la mise en oeuvre d'ondes guidées, non plus aux interfaces métal:diélectrique, mais autour de zone optiquement guidante à haut indice optique ;
- la mise en oeuvre d'ondes acoustiques de surface ;
- la mise en oeuvre de composants électroniques intégrés ;
- la mise en oeuvre de mesure d'impédance ;
- la mise en oeuvre de magnétorésistance ; ou encore
- la mise en oeuvre de micro-miroirs déflecteurs.

Ces méthodes sont bien connues de l'homme de l'art. On pourra par exemple se réferer aux documents suivants :
Dharuman et al., « Label-free impedance detection of oligonucleotide hybridisation on interdigitated ultramicroelectrodes using electrochemical redox probes », Biosens. and Bioelect. 21, 645-654, 2005 ;
Cheng et al., "An array-based CMOS biochip for electrical detection of DNA with multilayer self-assembly gold nanoparticles", Sensors and Actuators, B 109, 249-255, 2005 ;
Nikitin et al., "Picoscope, a new label-free biosensor", Sensors and Actuators B 111-112, 500-504, 2005 ; ou encore
dans le document de revue générale « Emerging tools for real-time label free detection of interactions on functional protein microarrays » de Ramachadran et al. (FEBS Journal (Federation of European Biochemical Society) volume 272, pages 5412 à 5425, 2005) ainsi qu'aux documents cités par référence dans ce document pour la mise en oeuvre de ces méthodes avec les supports qui leur sont adaptés.

Par comparaison avec la méthode SPR, les autres méthodes de lecture dynamiques sans marqueur citées également ci-avant ne nécessitent que des changements mineurs dans la fonctionnalisation des surfaces préalablement aux dépôts des sondes multi-fonctionnalisées, ceci notamment au support utilisé pour la méthode de lecture choisie. Les procédures ou protocoles à mettre en oeuvre pour leur utilisation sont identiques à ceux mis en oeuvre pour la méthode SPR (dont la lecture nécessite l'utilisation d'un film métallique).

Selon un mode de réalisation préféré, le procédé selon l'invention est caractérisé en ce que ledit support est un support solide transparent, de préférence une lame de verre, lorsque la mesure de la formation des complexes aux étapes b) et d) est effectuée par lecture d'un signal à la surface inférieure du support solide (opposée à la surface du support où les composés sondes sont fixés), en particulier lorsque le procédé selon l'invention est caractérisé en ce que la mesure de la formation des complexes aux étapes b) et d) est effectuée par imagerie par Surface Plasmon Résonance (« SPR »).

Parmi d'autres modes de réalisation du procédé selon l'invention, on peut citer les procédés dans lesquels le composé Cc et le composé Ccc sont marqués à l'aide d'un marqueur.

Lorsque le procédé de l'invention utilise un système de mesure nécessitant le marquage préalable des composés cibles (i.e. mesure d'un signal de fluorescence ou de radioactivité), ces composés cibles Cc et Ccc dont on cherche à détecter et/ou quantifier la présence sont préalablement marqués par un marqueur capable de générer directement ou indirectement un signal détectable, de préférence détectable par fluorescence.

De préférence, les composés Cc et Ccc sont préalablement marqués par un marqueur différent, notamment lorsque la mesure des complexes Cs/Cc est réalisée en présence du complexe Csc/Ccc ou vice versa.

Ainsi, selon un mode de réalisation préféré, le procédé selon l'invention est caractérisé en ce que ledit marqueur est un marqueur fluorescent.

De préférence, lesdits marqueurs lorsqu'il s'agit de marqueurs fluorescents sont choisis parmi des dérivés de la cyanine, de préférence choisis parmi les dérivés sulfonatés de la cyanine, notamment les composés Cy5 ou Cy3, avec les fluorochromes de la gamme. Alexa Fluor™ (Molecular Probes Inc., U.S.A), ou encore avec la rhodamine ou ses dérivées.

Selon un mode de réalisation préféré, le procédé selon l'invention est caractérisé en ce que lesdits marqueurs utilisés pour le marquage des composés Cc et Ccc sont identiques et caractérisé en ce que :
- lorsque les étapes c) et d) sont réalisées préalablement aux étapes a) et b), le support solide est lavé dans des conditions appropriées après l'étape d) afin d'éliminer du support solide le composé Ccc du complexe Csc/Ccc ; ou
- lorsque les étapes c) et d) sont réalisées postérieurement aux étapes a) et b), le support solide est lavé dans des conditions appropriées après l'étape b) afin d'éliminer du support solide le composé Cc du complexe Cs/Cc.

Selon un mode de réalisation préféré, le procédé selon l'invention est caractérisé en ce que lesdits marqueurs utilisés pour le marquage des composés Cc et Ccc sont différents.

Par exemple, lorsque les marqueurs sont de type fluorescent et que les composés Cc et Ccc sont des acides nucléiques, les composés Cc et Ccc pourront être respectivement marqués avec les marqueurs Cy3 et Cy5 ou vice versa.

Les marquages de composés nucléiques avec notamment des marqueurs fluorescents sont bien connus de l'homme de l'art et ne seront pas développés ici. On peut par exemple citer sans s'y limiter lorsque la cible recherchée est un ADN génomique la technique de marquage mettant en oeuvre une ADN polymérase (type enzyme de Klenow) pour la synthèse d'un brin complémentaire en présence de nucléotides marqués (par exemple Cy3/Cy5 dCTP), ou lorsque la cible recherchée est un ARN la technique de marquage indirecte mettant en oeuvre une transcriptase inverse (RT) en présence d'amino allyl dUTP.

Sous un autre aspect, la présente invention a pour objet l'utilisation d'un support solide comprenant fixés à sa surface supérieure un premier composé sonde Cs capable de se lier spécifiquement avec un composé cible Cc susceptible d'être contenu dans un échantillon, et un deuxième composé sonde Csc capable de se lier spécifiquement avec un autre composé cible Ccc, le rapport molaire entre ledit composé Csc et ledit composé Cs étant connu, ledit support solide étant destiné à la détermination et/ou à la quantification de la présence du composé cible Cc à l'aide d'un système permettant de mesurer la formation de complexe spécifique Cs/Cc par une méthode de lecture dynamique, de préférence sans marqueur, pour la calibration ou l'autocalibration de ladite mesure effectuée à la surface supérieure ou inférieure dudit support après mise en contact de l'échantillon susceptible de contenir le composé cible Cc sur la surface supérieure du support.

De préférence, l'utilisation d'un support solide selon l'invention est caractérisée en ce que ledit composé Csc et ledit composé Cs sont fixés indépendamment à la surface supérieure dudit support par un même procédé, de manière plus préférée en ce que ledit composé Csc est fixé à la surface supérieure dudit support par son couplage covalent l'une des extrémités du composé Cs.

Selon des modes de réalisation préférés, l'utilisation d'un support selon l'invention sera caractérisée en ce que ledit support et les composés Cs, Csc, Ccc et Cc possèdent en outre les caractéristiques telles que définies indépendamment dans le procédé de l'invention ci-avant décrit.

De préférence, l'utilisation d'un support solide selon l'invention est caractérisée en ce que ledit support est revêtu d'une couche métallique et en ce que la méthode de lecture dynamique sans marqueur utilisée est l'imagerie SPR.

Sous encore un autre aspect, la présente invention a pour objet un kit ou nécessaire pour la calibration ou l'autocalibration d'une mesure destinée à, ou kit ou nécessaire pour la détermination et/ou la quantification de la présence d'un composé cible Cc dans un échantillon à l'aide d'un système de mesure de la formation d'un complexe spécifique entre ledit composé Cc et un composé sonde Cs fixé à la surface supérieure d'un support solide, ledit composé Cs étant capable de se lier spécifiquement avec ledit composé cible Cc, caractérisé en ce que la mesure de la formation des complexes est réalisée par une méthode de lecture dynamique, de préférence sans marqueur, et en ce que ledit kit ou nécessaire comprend :
a)
   - un support solide sur lequel sont fixés à sa surface supérieure un composé sonde Cs et un composé sonde de calibration Csc différent dudit composé sonde Cs, le rapport molaire entre ledit composé Csc et ledit composé Cs étant connu, ou le cas échéant
   - un support solide, un réactif Rs comprenant le composé Cs et un réactif Rc comprenant le composé Csc, ces deux composés Cs et Csc étant destinés à être fixés à la surface supérieure dudit support dans un rapport molaire connu ; et
b) un réactif Rcc comprenant un composé cible de calibration Ccc, de préférence en concentration connue, le cas échéant sous forme sèche à reconstituer ou en solution, le composé Ccc étant capable de se lier spécifiquement avec ledit composé Csc.

De préférence, le kit ou nécessaire selon l'invention est caractérisé en ce que le composé Csc est couplé par liaison covalente à l'une des extrémités du composé Cs, de préférence à l'extrémité libre non fixée au support du composé Cs.

- De préférence les composés Cs et Csc sont des acides nucléiques.

Selon des modes de réalisation préférés, le kit ou nécessaire selon l'invention sera caractérisé en ce que ledit support et les composés Cs, Csc, Ccc et Cc possèdent en outre les caractéristiques telles que définies indépendamment dans le procédé de l'invention ci-avant décrit.

De préférence, le kit ou nécessaire selon l'invention est caractérisé en ce que ledit support est revêtu d'une couche métallique et en ce que la méthode de lecture dynamique sans marqueur utilisée est l'imagerie SPR.

Sous un dernier aspect, la présente invention comprend un dispositif pour la mesure de la formation spécifique d'un composé Cc sur un support solide sur lequel est fixé à sa surface supérieure un composé sonde Cs capable de se lier spécifiquement avec ledit composé cible Cc, caractérisé en ce qu'il comprend :
- ledit support solide comprenant en outre fixé sur sa surface supérieure un composé sonde de calibration Csc, différent du composé Cs, capable de se lier spécifiquement avec un composé cible de calibration Ccc, différent du composé Cc, le rapport molaire entre ledit composé Csc et ledit composé Cs étant connu, ledit composé Csc et ledit composé Cs pouvant être fixés indépendamment à la surface supérieure dudit support par un même procédé ou ledit composé Csc pouvant être fixé à la surface supérieure dudit support par couplage covalent l'une des extrémités du composé Cs ;
- un réactif comprenant un composé cible de calibration Ccc, le cas échéant sous forme sèche à reconstituer ou en solution, capable de se lier spécifiquement avec ledit composé Csc ; et
- un système de lecture par une méthode de lecture dynamique, de préférence sans marqueur, de la surface supérieure ou inférieure dudit support permettant de mesurer la formation des complexes spécifiques Cs/Cc et Csc/Ccc obtenus à la surface supérieure du support.

Selon des modes de réalisation préférés, le dispositif selon l'invention est caractérisé en ce que ledit support, les composés Cs, Csc, et Ccc et le système de mesure possèdent en outre les caractéristiques telles que définies indépendamment dans le procédé de l'invention ci-avant décrit.

De préférence, lé dispositif selon l'invention est caractérisé en ce que ledit support est revêtu d'une couche métallique et en ce que la méthode de lecture dynamique sans marqueur utilisée est l'imagerie SPR.

L'utilisation d'un kit ou dispositif selon l'invention dans le secteur de la santé pour le génotypage de mutations identifiées, pour l'obtention de profil comparée d'expression de gène, mais également par exemple dans le cadre du contrôle sanitaire en traçabilité et du contrôle qualité dans le cas des OGM (Organismes Génétiquement Modifiés) fait bien entendu partie de la présente invention.

D'autres caractéristiques et avantages de l'invention apparaissent dans la suite de la description avec les exemples et les figures dont les légendes sont représentées ci-après.

### LÉGENDES DES FIGURES

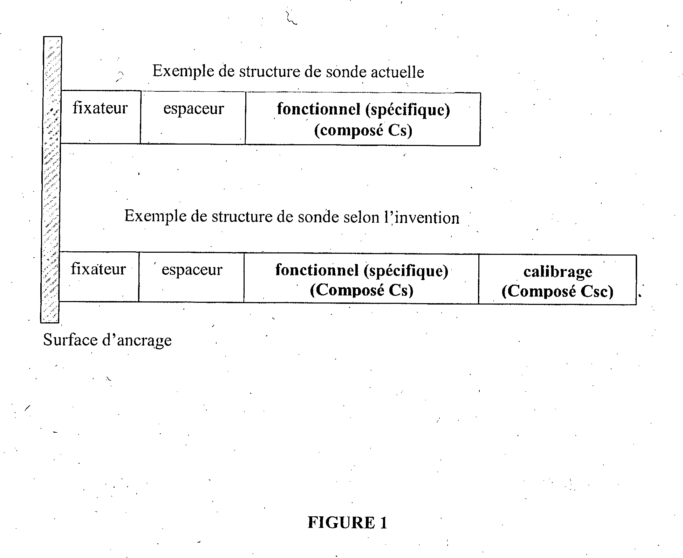

**Figure** 1 : La figure 1 schématise un exemple de structure d'une sonde multifonctionnelle selon l'invention représentée en unités fonctionnelles où la mesure locale de la concentration accessible effective du groupe de calibrage (Csc) est directement proportionnelle à concentration accessible effective du groupe fonctionnel spécifique (Cs).
**Figures 2A et 2B** **:** Exemple de 100 mesures supposément identiques mais dont les valeurs sont dispersées, notamment à cause de variations spatiales de concentrations en sondes accessibles. La figure 2B représente les valeurs ponctuelles obtenues pour les histogrammes représentés à la figure 2A.
**Figures 3A et 3B** **:** Histogramme associé aux figures 2A et 2B précédentes, les mesures brutes corrigées par les mesures de calibration sont significativement moins dispersées.
La figure 3B représente les valeurs ponctuelles obtenues pour les histogrammes représentés à la figure 3A.

### EXEMPLES

**Exemple 1 :** Réalisation d'un support solide pour imagerie SPR présentant fixé à sa surface une sonde Cs de nature nucléique spécifique d'un composé cible, couplée à une sonde de calibration Csc.

### Matériels et Méthodes

### • Exemple de fonctionnalisation adéquat dans le cas de biopuces dynamiques ADN/ADN

Nous avons réalisé des puces en partant de substrats de verre, du type lame de microscope, sur lequel a été déposée une couche de chrome, de l'ordre de 2 nm, et une couche d'or, de l'ordre de 50 nm. Sur ce dépôt métallique, un auto-assemblage moléculaire, du type MUA (acide 11-mercaptoundecanoic) / PEI (poly(ethylenimine) / extravidine a été réalisé tel que décrit dans le document Bassil et al., 2003 ou du type 11-mercaptoundecanol / Dextran/avidine (Biacore). La couche finale étant riche en groupement avidine, elle est particulièrement bien adaptée aux dépôts de nouveaux groupements fonctionnalisés par des biotines (le complexe avidine/biotine est particulièrement stable). Les biopuces ont été fonctionnalisées en spottant des séquences sondes biotinylées (les séquences sondes sont diluées à une concentration de 7 µM dans une solution de PBS 1X et de bétaïne 1,5 M). Tous les produits utilisés sont disponibles commercialement.

La séquence de calibrage Csc est dans cet exemple introduite par couplage covalent à l'extrémité 3' de la séquence sonde Cs pour l'ensemble des plots sondes.

Par exemple pour caractériser le génotype pour la mutation "MV470" de l'exon n 10 du gène CFTR lié à la mucoviscidose les structures de séquences suivantes ont été utilisées.

Mutations : accrochage (5' Biotin) + espaceur ((T)16) + séquences cible + calibration (gac cgg tat gcg) soit, pour le composé sonde Cs couplé au composé sonde de calibration Csc, respectivement pour le gène non muté (« Wild type ») M470V-WT et muté (« Mutated type ») M470V - MT et le contrôle négatif :

| | |
|---|---|
| M470V-WT | 5' Biotin (T)16 TTC TAA TGA TGA TTA gac cgg tat gcg 3' |
| M470V-MT | 5' Biotin (T)16 TTC TAA TGG TGA TTA gac cgg tat gcg 3' |
| contrôle négatif | 5' Biotin (T)18 CAC TTC GTG CCT T gac cgg tat gcg 3' |

Les séquences de calibrages peuvent être prises quelconques du moment que leurs longueurs soient telles que les duplex complémentaires formés sont de stabilité raisonnables et préférentiellement qu'elles ne présentent pas d'interactions croisées avec les autres séquences mises en oeuvre sur la biopuce. Pour simplifier le processus de choix d'une telle séquence sonde de calibration, celles-ci peuvent être sélectionnées parmi celles qui ont été introduites comme « zip » (voir le document Gerry et al., J. Mol. Biol., vol. 292, pp 251-262,1999) et qui ont été déterminées pour ne pas avoir a priori d'interactions avec les séquences présentes dans le génome humain. Ce type de séquence comme composé Csc a notamment pour avantage de pouvoir être utilisé sans devoir être modifié quel que soit le composé cible recherché.

Lors des expériences, réalisées en tampon PBS 1X (25 mM phosphate buffer with 0,137 M NaCl, pH 7,4), l'échantillon de composé cible de calibration Ccc est d'abord mis en contact avec la biopuce avant l'échantillon de composé cible Cc ou inversement, le duplex formé entre Csc et Ccc (ou inversement entre Cs et Cc) étant dénaturé entre les deux séries de mesures. Dans un autre mode de réalisation, il est possible de ne pas dénaturer le premier duplex formé entre Csc et Ccc (ou inversement entre Cs et Cc). Pour ce dernier mode de réalisation il est préféré de placer le composé sonde de calibration plutôt entre la surface et le composé sonde.

### • Le système de mesure utilisé

La lecture s'effectue par imagerie en mode de résonance de plasmons de surface (imagerie par SPR). La grandeur mesurée est un niveau de réflectivité, noté R, en polarisation TM (transverse magnétique) rapportée à une polarisation TE (transverse électrique). Ce phénomène physique nous permet d'avoir 6 dimensions associées à nos mesures : deux spatiales, notées x et y, une temporelle notée t, une spectrale notée λ, une angulaire notée θ et une de polarisation notée TMi. La condition de résonance est affectée par toute variation de l'indice optique n au voisinage de la surface, notamment celle induite par hybridation de molécules biochimiques, formant un film d'épaisseur moyenne notée e. La résolution sub-nanométrique est suffisante pour nombre d'applications, notamment celles nécessitant la détermination d'hybridation de séquences d'ADN.

Une telle mesure peut être effectuée en temps réel et permet de suivre l'évolution d'une surface dans le temps.

Dans le cas général de biopuces dynamiques, où plusieurs mesures peuvent être effectuées séquentiellement temporellement, il est possible d'associer à une étape de suivi d'interactions biomoléculaires d'intérêts, une étape de calibration qui va permettre de corriger la mesure expérimentale brute et tenant compte des fluctuations locales des conditions dans lesquelles ces mesures ont été effectuées.

Il s'agit d'accrocher avec la même spécificité potentielle que durant la mesure réelle une entité de calibrage. Ceci permet de quantifier pour tous les pixels (x, y, *) leur fonctionnalité réelle en mesurant les Δn/Δe qui leur sont associés. Il est alors possible de tenir compte des inhomogénéités effectives des surfaces de biopuces, notamment concernant les densités de sondes accessibles, pour interpréter et corriger quantitativement les dispersions observées dans leurs comportements durant les phases de mesures. De fait, cette correction affranchit les mesures de toutes les causes de variations spatiales stables dans le temps. Ceci permet ainsi, dans l'état actuel de la maîtrise relative des surfaces, de gagner plus d'un ordre de grandeur sur la précision des mesures. De façon générale, cela minimise les contraintes sur le cahier des charges des composants mis en jeu.

### Exemple 2 : Résultats

### A) Exemple de relevé de mesures obtenues sur 100 pixels d'un plot (ou 100 plots supposément identiques)

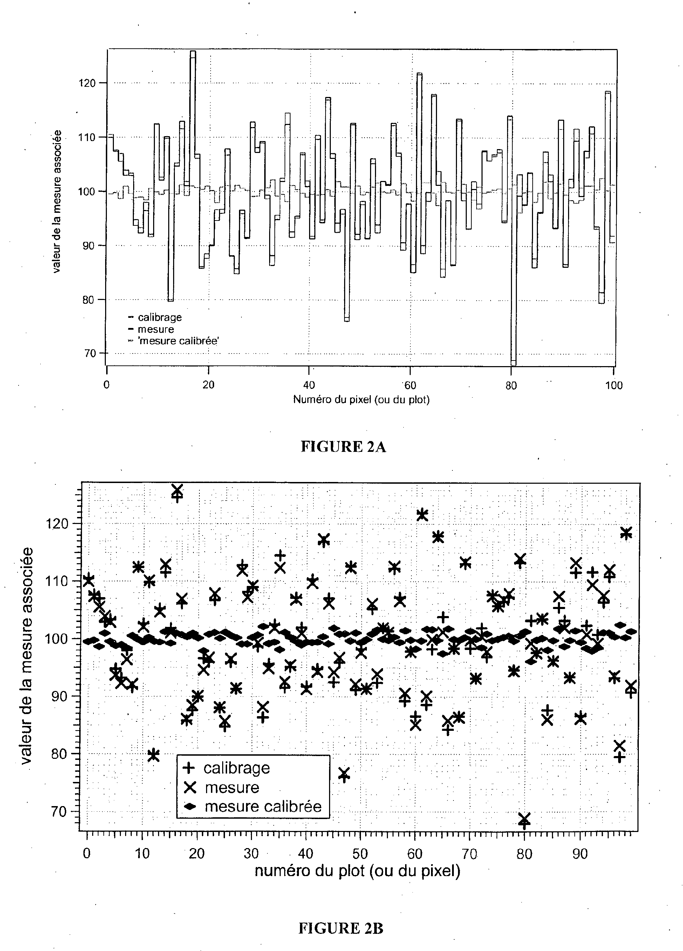

### Voir Figures 2A et 2B

Relevé de mesures (symbole X (*multiplie*)) sur 100 pixels d'un plot (ou 100 plots supposément identiques).

Pour les mêmes pixels (respectivement plots) les mesures correspondantes de calibrage sont représentées par des symbole + *(plus).*

Les données de mesures corrigées par les variations mesurées dans la phase de calibration sont représentées par les symboles ◆ (losange allongé horizontalement).

Dans le cas présenté, les valeurs des mesures de calibrage sont issues d'une répartition gaussienne centrée sur 100 et de largeur 10, celles de mesures sont corrélées aux mesures de calibrages avec une dispersion gaussienne de largeur 1. C'est l'ordre de grandeur de cette dernière largeur que nous retrouvons naturellement, seule, dans les données corrigées.

### B) Histogramme des mesures brutes corrigées par les mesures de calibration

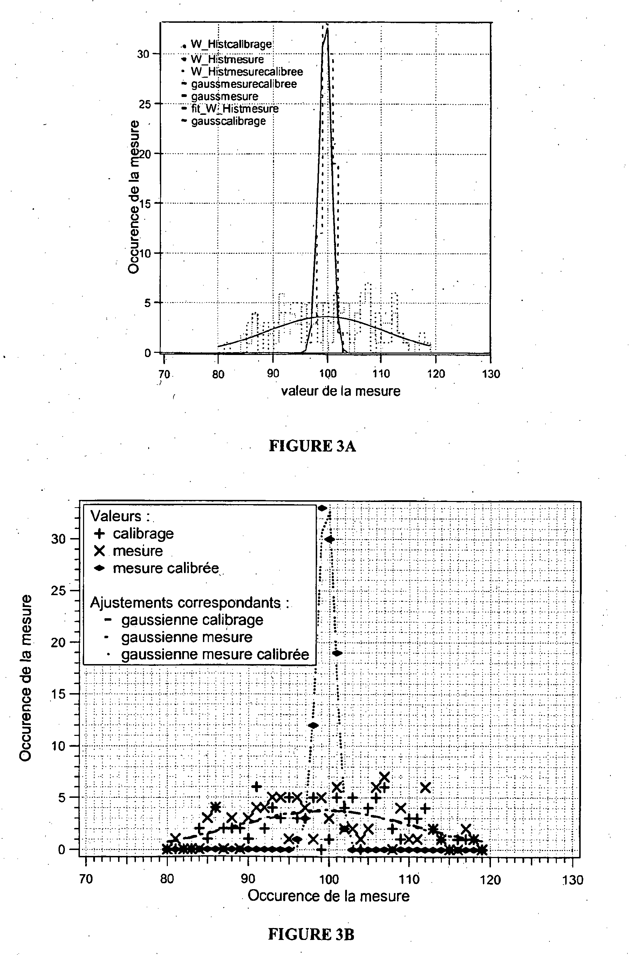

### Voir figures 3A et 3B

Les histogrammes correspondant à une telle sérié de mesures sur 100 pixels d'un plot (ou 100 plots supposément identiques) sont illustrés par les figures 3A et 3B.

Les mesures brutes corrigées par les mesures de calibration sont significativement moins dispersées.

## Revendications

1. Procédé de détermination par imagerie par résonance de plasmon de surface (« imagerie SPR ») de la présence et/ou de la quantité d'un composé cible Cc dans un échantillon à tester, ledit procédé mettant en oeuvre un support solide revêtu d'une couche métallique sur lequel est fixé à sa surface supérieure un composé sonde Cs capable de se lier spécifiquement avec ledit composé Cc susceptible d'être contenu dans l'échantillon à tester, ledit procédé comprenant :
- une étape a) de mise en contact de l'échantillon à tester susceptible de contenir ledit composé Cc avec ledit support dans des conditions permettant la formation spécifique du complexe Cc/Cs ; et
- une étape b) de mesure par imagerie SPR de la formation du complexe Cc/Cs éventuellement formé à l'étape a),
**caractérisé en ce que** :
- ledit support solide comprend en outre fixé à cette surface supérieure un composé sonde de calibration Csc capable de se lier spécifiquement avec un composé cible de calibration Ccc, le rapport molaire entre ledit composé Csc et ledit composé Cs étant connu, et les composés Cs et Csc étant fixés sur un même plot, et **en ce que** le procédé comprend préalablement ou postérieurement aux étapes a) et b) les étapes suivantes :
c) la mise en contact d'un échantillon dudit composé Ccc avec ledit support dans des conditions permettant la formation spécifique du complexe Csc/Ccc ; et
d) la mesure par imagerie SPRde la formation du complexe Csc/Ccc à l'étape c),
la détermination de la présence et/ou de la quantité dudit composé cible Cc dans l'échantillon à tester étant basée sur les mesures obtenues à l'étape b) et à l'étape d).

2. Procédé selon la revendication 1, **caractérisé en ce que** :
- lorsque les étapes c) et d) sont réalisées préalablement aux étapes a) et b), le support solide est lavé dans des conditions appropriées après l'étape d) afin d'éliminer du support solide le composé Ccc du complexe Csc/Ccc ; ou
- lorsque les étapes c) et d) sont réalisées postérieurement aux étapes a) et b), le support solide est lavé dans des conditions appropriées après l'étape b) afin d'éliminer du support solide le composé Cc du complexe Cs/Cc.

3. Procédé selon l'une des revendications 1 et 2, **caractérisé en ce qu'**un composé espaceur est fixé sur ledit support, et **en ce que** le composé Cs et le composé Cc sont fixés au support par l'intermédiaire dudit composé espaceur.

4. Procédé selon l'une des revendications 1 à 3, **caractérisé en ce que** le composé Csc est fixé à l'extrémité libre du composé Cs par liaison covalente.

5. Procédé selon l'une des revendications 1 à 4, **caractérisé en ce que** :
- les mêmes composés Cs et Csc sont fixés sur une même surface délimitée (dénommée « plot ») du support ;
- **en ce que** l'échantillon du composé Ccc et l'échantillon à tester susceptible de contenir le composé Cc à l'étape a) sont mis en contact avec tout ledit plot du support ;
- on effectue les mesures de l'étape b) et de l'étape d) pour un ensemble de points ou pixels dudit plot ; et
- la détermination de la présence et/ou de la quantité dudit composé cible Cc dans l'échantillon à tester est réalisée en tenant compte de l'ensemble des mesures obtenues à l'étape b) et à l'étape d) pour chacun des points ou pixels mesurés dudit plot.

6. Procédé selon la revendication 5, **caractérisé en ce que** pour un même plot, les conditions permettant la formation spécifique du complexe Cc/Cs et Ccc/Csc sont identiques.

7. Procédé selon l'une des revendications 5 et 6, **caractérisé en ce que** ledit support comprend n plots, n étant compris entre 2 et 10⁸.

8. Procédé selon l'une des revendications 5 à 7, **caractérisé en ce que** les composés Csc et Ccc utilisés sont identiques pour les n plots.

9. Procédé selon l'une des revendications 1 à 8, **caractérisé en ce que** ledit composé Cs et ledit composé Cc sont des acides nucléiques.

10. Procédé selon la revendication 9, **caractérisé en ce que** les composés Csc et Ccc sont des acides nucléiques ne présentant aucune homologie significative avec le composé Cs fixé et le composé Cc recherché, ou leur séquence complémentaire.

11. Procédé selon la revendication 9 ou 10, **caractérisé en ce que** le composé Csc et, le cas échéant, le composé Ccc, est un acide nucléique de longueur compris entre 6 et 30 nucléotides.

12. Procédé selon l'une des revendications 1 à 11, **caractérisé en ce que** ledit support est un support solide transparent, de préférence une lame de verre.

13. Utilisation d'un support solide revêtu d'une couche métallique comprenant fixés à sa surface supérieure un premier composé sonde Cs capable de se lier spécifiquement avec un composé cible Cc susceptible d'être contenu dans un échantillon, et un deuxième composé sonde Csc capable de se lier spécifiquement avec un autre composé cible Ccc, le rapport molaire entre ledit composé Csc et ledit composé Cs étant connu, les composés Cs et Csc étant fixés sur un même plot, ledit support solide étant destiné à la détermination et/ou à la quantification par imagerie SPR de la présence du composé cible Cc en mesurant la formation de complexe spécifique Cs/Cc, pour la calibration ou l'auto-calibration de ladite mesure après mise en contact de l'échantillon susceptible de contenir le composé cible Cc sur la surface supérieure du support.

14. Utilisation d'un support solide selon la revendication 13, **caractérisée en ce que** ledit composé Csc est fixé par couplage covalent à l'une des extrémités du composé Cs.

15. Kit ou nécessaire pour la calibration d'une mesure destinée à, ou kit ou nécessaire pour la détermination et/ou la quantification de la présence d'un composé cible Cc dans un échantillon par imagerie SPR par mesure de la formation d'un complexe spécifique entre ledit composé Cc et un composé sonde Cs fixé à la surface supérieure d'un support solide, ledit composé Cs étant capable de se lier spécifiquement avec ledit composé cible Cc, **caractérisé en ce que** ledit kit ou nécessaire comprend :
a)
- un support solide revêtu d'une couche métallique sur lequel sont fixés à sa surface supérieure un composé sonde Cs et un composé sonde de calibration Csc différent dudit composé sonde Cs, le rapport molaire entre ledit composé Csc et ledit composé Cs étant connu, les composés Cs et Csc étant fixés sur un même plot ou le cas échéant
- un support solide revêtu d'une couche métallique, un réactif Rs comprenant le composé Cs et un réactif Rc comprenant le composé Csc, ces deux composés Cs et Csc étant destinés à être fixés à la surface supérieure dudit support dans un rapport molaire connu les composés Cs et Csc étant fixés sur un même plot; et
b) un réactif Rcc comprenant un composé cible de calibration Ccc, de préférence en concentration connue, le composé Ccc étant capable de se lier spécifiquement avec ledit composé Csc.

16. Kit ou nécessaire selon la revendication 15, **caractérisé en ce que** le composé Csc est couplé par liaison covalente à l'une des extrémités du composé Cs.

17. Dispositif pour la mesure par imagerie SPR de la formation spécifique d'un composé Cc sur un support solide revêtu d'une couche métallique sur lequel est fixé à sa surface supérieure un composé sonde Cs capable de se lier spécifiquement avec ledit composé cible Cc, **caractérisé en ce qu'**il comprend :
- ledit support solide comprenant en outre fixé sur sa surface supérieure un composé sonde de calibration Csc, différent du composé Cs, capable de se lier spécifiquement avec un composé cible de calibration Ccc, différent du composé Cc, le rapport molaire entre ledit composé Csc et ledit composé Cs étant connu, et ledit composé Csc est fixé à la surface supérieure dudit support par couplage covalent à l'une des extrémités du composé Cs ;
- un réactif comprenant un composé cible de calibration Ccc capable de se lier spécifiquement avec ledit composé Csc ; et
- un système de lecture d'imagerie SPR dudit support permettant de mesurer la formation des complexes spécifiques Cs/Cc et Csc/Ccc obtenus à la surface supérieure du support.

## Patentansprüche

1. Verfahren zur Bestimmung mittels Oberflächenplasmonen-Resonanz-Bildgebung ("OPR-Bildgebung") der Anwesenheit und/oder der Menge einer Zielverbindung Cc in einer zu testenden Probe, wobei das Verfahren einen festen, mit einer Metallschicht überzogenen Träger einsetzt, auf dem auf seiner oberen Oberfläche eine Sondenverbindung Cs angebracht ist, die sich spezifisch mit der Verbindung Cc verbinden kann, die möglicherweise in der zu testenden Probe enthalten ist, wobei das Verfahren umfasst:
- einen Schritt a) des In-Kontakt-Bringens der zu testenden Probe, die möglicherweise die Verbindung Cc enthält, mit dem Träger unter Bedingungen, welche die spezifische Bildung des Komplexes Cc/Cs ermöglichen; und
- einen Schritt b) des Messens der Bildung des Komplexes Cs/Cs, der sich gegebenenfalls im Schritt a) gebildet hat, mittels OPR-Bildgebung,
**dadurch gekennzeichnet, dass**:
- der feste Träger darüber hinaus eine auf dieser oberen Oberfläche angebrachte Kalibrierungs-Sonden-Verbindung Csc umfasst, die in der Lage ist, sich spezifisch mit einer Kalibrierungs-Zielverbindung Ccc zu verbinden, wobei das Molverhältnis zwischen der Verbindung Csc und der Verbindung Cs bekannt ist und die Verbindungen Cs und Csc auf ein und derselben Versuchsfläche angebracht sind, und dadurch, dass das Verfahren vor oder nach den Schritten a) und b) die folgenden Schritte umfasst:
c) In-Kontakt-Bringen einer Probe der Verbindung Ccc mit dem Träger unter Bedingungen, welche die spezifische Bildung des Komplexes Csc/Ccc ermöglichen; und
d) Messen der Bildung des Komplexes Csc/Ccc im Schritt c) mittels OPR-Bildgebung, wobei die Bestimmung der Anwesenheit und/oder der Menge der Zielverbindung Cc in der zu testenden Probe auf den im Schritt b) und im Schritt d) erhaltenen Messungen beruht.

2. Verfahren nach Anspruch 1, **dadurch gekennzeichnet, dass**:
- wenn die Schritte c) und d) vor den Schritten a) und b) durchgeführt werden, der feste Träger nach dem Schritt d) unter geeigneten Bedingungen gewaschen wird, um die Verbindung Ccc des Komplexes Csc/Ccc von dem festen Träger zu entfernen; oder
- wenn die Schritte c) und d) nach den Schritten a) und b) durchgeführt werden, der feste Träger nach dem Schritt b) unter geeigneten Bedingungen gewaschen wird, um die Verbindung Cc des Komplexes Cs/Cc von dem festen Träger zu entfernen.

3. Verfahren nach einem der Ansprüche 1 und 2, **dadurch gekennzeichnet, dass** eine Abstandshalter-Verbindung auf dem Träger angebracht ist und dass die Verbindung Cs und die Verbindung Cc durch Zwischenschaltung der Abstandshalter-Verbindung an dem Träger angebracht sind.

4. Verfahren nach einem der Ansprüche 1 bis 3, **dadurch gekennzeichnet, dass** die Verbindung Csc durch kovalente Bindung am freien Ende der Verbindung Cs angebracht ist.

5. Verfahren nach einem der Ansprüche 1 bis 4, **dadurch gekennzeichnet, dass**:
- die selbigen Verbindungen Cs und Csc auf ein und derselben begrenzten Oberfläche (als "Versuchsfläche" bezeichnet) des Trägers angebracht sind; und
- dass die Probe der Verbindung Ccc und die zu testende Probe, welche die Verbindung Cc möglicherweise enthält, im Schritt a) mit der ganzen Versuchsfläche des Trägers in Kontakt gebracht werden;
- man die Messungen des Schrittes b) und des Schrittes d) für eine Menge von Punkten oder Pixeln der Versuchsfläche durchführt; und
- die Bestimmung der Anwesenheit und/oder der Menge der Zielverbindung Cc in der zu testenden Probe durchgeführt wird, indem man die Gesamtheit der Messungen, die im Schritt b) und im Schritt d) für jeden der von der Versuchsfläche gemessenen Punkte oder Pixel erhalten wurden, berücksichtigt.

6. Verfahren nach Anspruch 5, **dadurch gekennzeichnet, dass** bei ein und derselben Versuchsfläche die Bedingungen, welche die spezifische Bildung des Komplexes Cc/Cs und Ccc/Csc ermöglichen, identisch sind.

7. Verfahren nach einem der Ansprüche 5 und 6, **dadurch gekennzeichnet, dass** der Träger n Versuchsflächen umfasst, wobei n 2 bis 10⁸ beträgt.

8. Verfahren nach einem der Ansprüche 5 bis 7, **dadurch gekennzeichnet, dass** die verwendeten Verbindungen Csc und Ccc für die n Versuchsflächen identisch sind.

9. Verfahren nach einem der Ansprüche 1 bis 8, **dadurch gekennzeichnet, dass** die Verbindung Cs und die Verbindung Cc Nukleinsäuren sind.

10. Verfahren nach Anspruch 9, **dadurch gekennzeichnet, dass** die Verbindungen Csc und Ccc Nukleinsäuren sind, die keinerlei signifikante Homologie mit der angebrachten Verbindung Cs und der gesuchten Verbindung Cc oder ihrer komplementären Sequenz aufweisen.

11. Verfahren nach Anspruch 9 oder 10, **dadurch gekennzeichnet, dass** die Verbindung Csc und gegebenenfalls die Verbindung Ccc eine Nukleinsäure mit einer Länge zwischen 6 und 30 Nukleotiden einschließlich ist.

12. Verfahren nach einem der Ansprüche 1 bis 11, **dadurch gekennzeichnet, dass** der Träger ein fester transparenter Träger, vorzugsweise eine Glasplatte, ist.

13. Verwendung eines festen, mit einer Metallschicht überzogenen Trägers, der eine auf seiner oberen Oberfläche angebrachte erste Sondenverbindung Cs, die sich spezifisch mit einer Zielverbindung Cc verbinden kann, welche möglicherweise in einer Probe enthalten ist, und eine zweite Sondenverbindung Csc umfasst, die sich spezifisch mit einer anderen Zielverbindung Ccc verbinden kann, wobei das Molverhältnis zwischen der Verbindung Csc und der Verbindung Cs bekannt ist, wobei die Verbindungen Cs und Csc auf ein und derselben Versuchsfläche angebracht sind, wobei der feste Träger für die Bestimmung und/oder Quantifizierung der Anwesenheit der Zielverbindung Cc mittels OPR-Bildgebung durch Messen der Bildung des spezifischen Komplexes Cs/Cc bestimmt ist, für die Kalibrierung oder Auto-Kalibrierung der Messung nach In-Kontakt-Bringen der Probe, welche die Zielverbindung Cc möglicherweise enthält, auf der oberen Oberfläche des Trägers.

14. Verwendung eines festen Trägers nach Anspruch 13, **dadurch gekennzeichnet, dass** die Verbindung Csc durch kovalente Kupplung an einem der äußeren Enden der Verbindung Cs angebracht ist.

15. Kit oder Necessaire für die Kalibrierung einer Messung, die bestimmt ist für, oder Kit oder Necessaire für die Bestimmung und/oder Quantifizierung der Anwesenheit einer Zielverbindung Cc in einer Probe mittels OPR-Bildgebung durch Messen der Bildung eines spezifischen Komplexes zwischen der Verbindung Cc und einer Sondenverbindung Cs, die auf der oberen Oberfläche eines festen Trägers angebracht ist, wobei die Verbindung Cs sich spezifisch mit der Zielverbindung Cc verbinden kann, **dadurch gekennzeichnet, dass** das Kit oder Necessaire umfasst:
a)
- einen festen, mit einer Metallschicht überzogenen Träger, auf dem eine Sondenverbindung Cs und eine von der Sondenverbindung Cs verschiedene Kalibrierungs-Sondenverbindung Csc auf seiner oberen Oberfläche angebracht sind, wobei das Molverhältnis zwischen der Verbindung Csc und der Verbindung Cs bekannt ist, wobei die Verbindungen Cs und Csc auf ein und derselben Versuchsfläche angebracht sind, oder gegebenenfalls
- einen festen, mit einer Metallschicht überzogenen Träger, ein Reagens Rs, das die Verbindung Cs umfasst, und ein Reagens Rc, das die Verbindung Csc umfasst, wobei die beiden Verbindungen Cs und Csc dazu bestimmt sind, auf der oberen Oberfläche des Trägers in einem bekannten Molverhältnis angebracht zu werden, wobei die Verbindungen Cs und Csc auf ein und derselben Versuchsfläche angebracht werden; und
b) ein Reagens Rcc, das eine Kalibrierungs-Zielverbindung Ccc vorzugsweise in bekannter Konzentration umfasst, wobei die Verbindung Ccc in der Lage ist, sich spezifisch mit der Verbindung Csc zu verbinden.

16. Kit oder Necessaire nach Anspruch 15, **dadurch gekennzeichnet, dass** die Verbindung Csc durch kovalente Bindung an eines der äußeren Enden der Verbindung Cs gekuppelt ist.

17. Vorrichtung für die Messung mittels OPR-Bildgebung der spezifischen Bildung einer Verbindung Cc auf einem festen Träger, der mit einer Metallschicht überzogen ist, auf welcher auf deren oberen Oberfläche eine Sondenverbindung Cs angebracht ist, die sich spezifisch mit der Zielverbindung Cc verbinden kann, **dadurch gekennzeichnet, dass** sie umfasst:
- den festen Träger, der darüber hinaus eine auf seiner oberen Oberfläche angebrachte, von der Verbindung Cs verschiedene Kalibrierungs-Sondenverbindung Csc umfasst, die sich spezifisch mit einer von der Verbindung Cc verschiedenen Kalibrierungs-Zielverbindung Ccc verbinden kann, wobei das Molverhältnis zwischen der Verbindung Csc und der Verbindung Cs bekannt ist und die Verbindung Csc durch kovalente Kupplung an eines der äußeren Enden der Verbindung Cs auf der oberen Oberfläche des Trägers angebracht ist;
- ein Reagens, das eine Kalibrierungs-Zielverbindung Ccc umfasst, die sich spezifisch mit der Verbindung Csc verbinden kann; und
- ein System zur Ablesung der OPR-Bildgebung des Trägers, welches das Messen der Bildung der spezifischen Komplexe Cs/Cc und Csc/Ccc ermöglicht, die auf der oberen Oberfläche des Trägers erhalten werden.

## Claims

1. Method for determining by surface plasmon resonance imaging ("SPR imaging") the presence and/or quantity of a target compound Cc in a sample to be tested, said process using a solid support coated with a metal layer to whose upper surface is attached a probe compound Cs capable of binding specifically to said compound Cc likely to be contained in the sample to be tested, said method comprising:
a step a) of contacting the test sample likely to contain said compound Cc with said support under conditions allowing the specific formation of the complex Cc/Cs; and
a step b) of measuring by SPR imaging the formation of complex Cc/Cs possibly formed in step a),
**characterized in that**:
said solid support also includes attached to this upper surface a calibration probe compound Csc capable of binding specifically to a calibration target compound Ccc, the molar ratio of said compound Csc to said compound Cs being known, and compounds Cs and Csc being attached to a same plot, and **in that** the method comprises the following steps prior to or after steps a) and b):
c) contacting a sample of said compound Ccc with said support under conditions allowing the specific formation of complex Csc/Ccc; and
d) measuring by SPR imaging the formation of complex Csc/Ccc in step c),
the determination of the presence and/or quantity of said target compound Cc in the sample to be tested being based on the measurements obtained in step b) and step d).

2. Method according to claim 1, **characterized in that**:
when steps c) and d) are carried out prior to steps a) and b), the solid support is washed under appropriate conditions after step d) in order to eliminate compound Ccc of complex Csc/Ccc from the solid support; or
when steps c) and d) are carried out after steps a) and b), the solid support is washed under appropriate conditions after step b) in order to eliminate compound Cc of complex Cs/Cc from the solid support.

3. Method according to one of claims 1 and 2, **characterized in that** a spacer compound is attached to said support, and **in that** compound Cs and compound Cc are attached to the support by means of said spacer compound.

4. Method according to one of claims 1 to 3, **characterized in that** compound Csc is attached to the free extremity of compound Cs by means of a covalent bond.

5. Method according to one of claims 1 to 4, **characterized in that**:
the same compounds Cs and Csc are attached to a defined surface (called a "plot") of the support;
**in that** the sample of compound Ccc and the sample to be tested likely to contain compound Cc of step a) are contacted with the whole of said plot of the support;
measurements in steps b) and d) are carried out for a set of points or pixels of said plot; and
determination of the presence and/or quantity of said target compound Cc in the test sample is carried out taking into account the set of measurements obtained in steps b) and d) for each of the measured points or pixels of said plot.

6. Method according to claim 5, **characterized in that** for a same plot, the conditions allowing the specific formation of complex Cc/Cs and Ccc/Csc are identical.

7. Method according to one of claims 5 and 6, **characterized in that** said support comprises n plots, n being between 2 and 10⁸.

8. Method according to one of claims 5 to 7, **characterized in that** compounds Csc and Ccc used are identical for the n plots.

9. Method according to one of claims 1 to 8, **characterized in that** said compound Cs and said compound Cc are nucleic acids.

10. Method according to claim 9, **characterized in that** compounds Csc and Ccc are nucleic acids with no significant homology with compound Cs attached and compound Cc investigated or their complementary sequence.

11. Method according to claim 9 or 10, **characterized in that** compound Csc and, if need be, compound Ccc, is a nucleic acid with a length of 6 to 30 nucleotides.

12. Method according to one of claims 1 to 11, **characterized in that** said support is a transparent solid support, preferably a glass slide.

13. Use of a solid support coated with a metal layer comprising, attached to its upper surface, a first probe compound Cs capable of binding specifically to a target compound Cc likely to be contained in the sample, and a second probe compound Csc capable of binding specifically to another target compound Ccc, the molar ratio of said compound Csc to said compound Cs being known, compounds Cs and Csc being attached to a same plot, said solid support being intended to determining and/or quantifying by SPR imaging the presence of a target compound Cc by measuring the formation of a specific complex Cs/Cc, for calibration or self-calibration of said measurement after contacting the sample likely to contain the target compound Cc on the upper surface of the support.

14. Use of a solid support according to claim 13, **characterized in that** said compound Csc is attached to one of the extremities of compound Cs by covalent coupling.

15. Kit or necessary equipment for calibration of a measurement for, or kit or necessary equipment for determination and/or quantification of the presence of a target compound Cc in a sample by SPR imaging by measurement of the formation of a specific complex between said compound Cc and a probe compound Cs attached to the upper surface of a solid support, said compound Cs being capable of binding specifically to said target compound Cc, **characterized in that** said kit or necessary equipment comprises:
a) a solid support coated with a metal layer to which is attached at the upper surface a probe compound Cs and a calibration probe compound Csc different from said probe compound Cs, the molar ratio of said compound Csc to said compound Cs being known, compounds Cs and Csc being attached to a same plot, or if necessary
a solid support coated with a metal layer, a reagent Rs comprising compound Cs and a reagent Rc comprising compound Csc, these two compounds Cs and Csc being intended to be attached to the upper surface of said support in a known molar ratio, compounds Cs and Csc being attached to a same plot; and
b) a reagent Rcc comprising a calibration target compound Ccc, preferably of known concentration, compound Ccc being capable of binding specifically to said compound Csc.

16. Kit or necessary equipment according to claim 15, **characterized in that** compound Csc is coupled by covalent bonds to one of the extremities of compound Cs.

17. Device for measurement by SPR imaging of the specific formation of a compound Cc on a solid support coated with a metal layer to which is attached at the upper surface a probe compound Cs capable of binding specifically to said target compound Cc, **characterized in that** it includes:
said solid support also comprising attached to its upper surface a calibration probe compound Csc, different from compound Cs, capable of binding specifically to a calibration target compound Ccc, different from compound Cc, the molar ratio of said compound Csc to said compound Cs being known, and said compound Csc being attached to the upper surface of said support by covalent coupling to one of the extremities of compound Cs;
a reagent comprising a calibration target compound Ccc capable of binding specifically to said compound Csc; and
a reading system using SPR imaging of said support allowing measurement of the formation of specific complexes Cs/Cc and Csc/Ccc obtained at the upper surface of the support.
